# NEUE EUROPÄISCHE PATENTSCHRIFT

(11) **EP 1 455 803 B2**
(45) Veröffentlichungstag und Bekanntmachung des Hinweises auf die Entscheidung über den Einspruch: **23.03.2016**
(45) Hinweis auf die Patenterteilung: 27.07.2005
(21) Anmeldenummer: 02794984.1
(22) Anmeldetag: 11.12.2002
(51) Int. Cl.: A61K 31/728, A61K 31/16, A61P 27/02

(54) **VERWENDUNG VON PANTHENOL UND/ODER PANTHOTHENSÄURE UND HYALURONSÄURE/UND ODER HYALURONAT ZUR HERSTELLUNG EINER PHARMAZEUTISCHEN ZUSAMMENSETZUNG ZUR OPHTALMOLOGISCHEN ANWENDUNG**
USE OF PANTHENOL AND/OR PANTOTHENIC ACID AND HYALURONIC ACID AND/OR HYALURONATE FOR THE MANUFACTURE OF A PHARMACEUTICAL COMPOSITION FOR USE IN OPHTHALMOLOGY
UTILISATION DU PANTHENOL ET/OU D'ACIDE PANTOTHÉNIQUE OU D'ACIDE HYALURONIQUE ET/OU DE HYALURONATE POUR LA MANUFACTURE D'UNE COMPOSITION PHARMACEUTIQUE POUR UNE UTILISATION OPHTALMOLOGIQUE

(30) Priorität: 12.12.2001 DE 10161110
(43) Veröffentlichungstag der Anmeldung: 15.09.2004
(73) Patentinhaber: URSAPHARM Arzneimittel GmbH & Co. KG, 66129 Saarbrücken (DE)
(72) Erfinder: GROSS, Dorothea, 66386 St. Ingbert (DE); HOLZER, Frank, 66386 St. Ingbert (DE)
(74) Vertreter: Pfenning, Meinig & Partner mbB
(86) Internationale Anmeldenummer: PCT/DE2002/004527
(87) Internationale Veröffentlichungsnummer: WO 2003/049747

(56) Entgegenhaltungen:
- EP-A1- 0 366 888
- EP-A2- 0 773 022
- WO-A-02/60495
- DE-A- 19 923 829
- DE-A1- 4 304 893
- DE-A1- 19 541 919
- US-A- 4 851 521
- CONDON P.I. ET AL: 'Double blind, randomised, placebo controlled, crossover, multicentre study to determine the efficacy of a 0.1% (w/v) sodium hyaluronate solution (Fermavisc) in the treatment of dry eye syndrome' J. OPHTHALMOL Bd. 83, 1999, Seiten 1121 - 1124
- 'Rote Liste 2000 Arzneimittelverzeichnis für Deutschland (einschließlich EU-Zulassungen)', 2000, EDITIO CANTOR VERLAG, AULENDORF Seiten 72-004 - 72-121

## Beschreibung

Die Erfindung betrifft die Verwendung von Panthenol und/oder Panthothensäure und Hyaluronsäure und/oder Hyaluronat zur Herstellung einer pharmazeutischen Zusammensetzung.

Das Krankheitsbild des "Trockenen Auges" wird auch als Sicca-Syndrom oder auch als Sicca-Symptomatik bezeichnet. Das Krankheitsbild des "Trockenen Auges", dessen Symptome sich unter anderem in Brennen, Kratzen, Sandkorngefühl im Auge und verschwommenen Sehen äußern, ist auf funktionelle Störungen des Tränenfilms zurückzuführen.

Das Krankheitsbild des "Trockenen Auges" kann auch auf einen verminderten Tränenfluß zurückzuführen sein, der vielfältige pathologischen Ursachen haben kann. Aufgrund des verminderten Tränenflusses kann sich nur ein ungenügender oder gar kein Tränenfilm auf der Augenoberfläche ausbilden. Der Tränenfilm wirkt unter anderem als Schmier- oder Gleitmittel zwischen Augenlid und Augenoberfläche. Aufgrund des fehlenden oder ungenügenden Tränenfilms kann es zu erheblichem Trauma der epithelialen Schichten führen.

Als Ursache für diese funktionellen Störungen kommen beispielsweise auch Allergien hervorrufende Umwelteinflüsse, wie beispielsweise Schadstoff- oder Ozonbelastung, in Frage. Insbesondere die in Sommermonaten ansteigende Ozonbelastung kann nicht nur eine Störung der Tränenproduktion verursachen, sondern auch eine Störung des physiologischen Tränenfilms. Beispielsweise werden in natürlichem Tränenfilm enthaltene Proteine und Hyaluronsäure durch Einwirkung von Ozon zerstört. Weiterhin hat sich gezeigt, daß das Sicca-Syndrom häufig mit einer durch Kosmetika am Auge hervorgerufenen Kontaktallergie verbunden ist.

Aus Spektrum Augenheilkunde (1998) 3/4: 174-176 ist bekannt, daß hypoosmolare Natrium-Hyaluronat-Tropfen zur Therapie des Krankheitsbildes des "Trockenen Auges" verwendet werden können. Dabei wurde Natrium-Hyaluronat mit einem Molekulargewicht von 5.000.000 Dalton verwendet.

Weiterhin ist aus Spektrum Augenheilkunde (1995) 9/5: 215-217 bekannt, daß bakteriell synthetisiertes Hyaluronat zur Behandlung des Krankheitsbildes des "Trockenen Auges" verwendet werden kann. Aus Jpn. J. Ophthalmol. (1996) 40: 62-65, ist bekannt, daß die Verbesserung der Tränenfilmstabilität durch Natrium-Hyaluronat-Augentropfen erreicht wird, die mindestens 0,1 % Natriumhyaluronat enthalten.

Aus Klinische Monatsblätter für Augenheilkunde (1996) 209: 84-88 ist bekannt, dass Dexpanthenol-haltige Augentropfen die corneaepitheliale Barrierefunktion, die aufgrund eines insuffizienten Tränenfilms gestört ist, bessern.

Die Verwendung von Panthenol ist weiterhin zur Behandlung von Verätzungen, Verbrennungen und Strahlenschäden der Haut sowie zur Behandlung von Entzündungen der Augen bekannt.

Aus der DE 19923829 ist eine Zusammensetzung von Natriumhyaluronat und Panthenol zum Einsatz in Kosmetika bekannt. Die US 4,851,521 offenbart Hyaluronsäureester zur medizinischen Verwendung.

Aufgabe der vorliegenden Erfindung ist es, eine bessere Therapie von Erkrankungen des Auges, die insbesondere mit funktionellen Störungen des Tränenfilms oder mit einem verminderten Tränenfluß einhergehen, zu ermöglichen.

Die Aufgabe wird durch die Verwendung von Panthenol und/oder Panthothensäure und Hyaluronsäure und/oder Hyaluronat sowie gegebenenfalls zusätzlichen pharmazeutischen Hilfsmitteln zur Herstellung einer pharmazeutischen Zusammensetzung zur Behandlung ophtalmologischer Fehlfunktionen gelöst.

Unter "pharmazeutischen Hilfsmitteln" werden Lösungsmittel, Lösungsvermittler, Lösungsbeschleuniger, Salzbildner, Salze, Puffersubstanzen, Viskositäts- und Konsistenzbeeinflusser, Gelbildner, Emulgatoren, Solubilisatoren, Benetzer, Spreizmittel, Antioxidantien, Konservierungsmittel, Füll- und Trägerstoffe, etc. verstanden.

Panthenol, d.h. (R,S)-2,4-Dihydroxy-N-(3-hydroxypropyl)-3,3-dimethylbutyramid, das auch als Pantothenol bzw. Pantothenylalkohol bezeichnet wird, ist als Epithelialisierungsmittel der Haut bekannt. Panthenol ist das alkoholische Analogon der Pantothensäure und besitzt aufgrund der intermediären Umwandlung die gleiche biologische Wirksamkeit wie die Pantothensäure.

Es hat sich herausgestellt, daß die bei der erfindungsgemäßen Verwendung hergestellte pharmazeutische Zusammensetzung zur Therapie ophthalmologischer Fehlfunktionen verwendbar ist.

Die Erfinder der vorliegenden Erfindung haben weiterhin überraschend herausgefunden, daß es bei der gleichzeitigen Applikation von Panthenol und/oder Pantothensäure und Hyaluronsäure und/oder Hyaluronat am Auge bzw. auf der Augenoberfläche zu einer synergistischen Wirkung bei der Behandlung von ophthalmologischen Erkrankungen, die mit funktionalen Störungen des Tränenfilms oder einem ungenügend ausgebildeten Tränenfilm verbunden sind, kommt.

Insbesondere hat sich gezeigt, daß es zu einer beschleunigten Epithelialisierung einer aufgrund eines insuffizienten Tränenfilms beschädigten Hornhaut bei topischer Anwendung der pharmazeutischen Zusammensetzung kommt.

Bevorzugt wird die bei der erfindungsgemäßen Verwendung hergestellte pharmazeutische Zusammensetzung bei der Behandlung ophthalmologischer Fehlfunktionen in Form von Augentropfen, Augenlösungen, Augenwässer, Augensprays, Augensalben oder Augentabletten zur topischen Anwendung am Auge oder auf der Augenoberfläche bereitgestellt.

Zur Herstellung einer Augensalbe kann Hyaluronsäure und/oder Hyaluronat und wenigstens Panthenol und/oder Pantothensäure beispielsweise in eine Mischung aus dickflüssigem Paraffin und weißem Vaselin eingebracht werden. Weiterhin kann in Salben beispielsweise auch dünnflüssiges Paraffin oder Wollwachs verwendet werden.

Bevorzugt wird die pharmazeutische Zusammensetzung in Form eines Augensprays oder in Form von Augentropfen bereitgestellt. In der Regel wird dabei die Hyaluronsäure und/oder das Hyaluronat sowie das Panthenol und/oder die Pantothensäure in wäßrigen Lösungen aufgelöst.

Die wäßrigen Lösungen sind dabei gemäß einer bevorzugten Ausführungsform zur ophthalmologischen Anwendung, bezogen auf die Tränenflüssigkeit, isotone Lösungen. Bei isotonen Lösungen liegt die Osmolarität etwa bei 300 mOsm/l. Gemäß einer weiteren bevorzugten Ausführungsform ist die pharmazeutische Zusammensetzung hypoosmolar. In diesem Fall kann die Osmolarität beispielsweise etwa 160-180 mOsm/l betragen. Eine hypoosmolare Lösung findet insbesondere dann Anwendung, wenn eine abnorm hohe Osmolarität eines Tränenfilms bei einem Patienten mit trockenen Augen ausgeglichen werden muß.

Zur Isotonisierung der wäßrigen Lösung werden Natriumchlorid, Borsäure, etc. verwendet. Der pH-Wert der wäßrigen Lösung liegt in einem Bereich von pH 6 bis pH 9, bevorzugt pH 6,5 bis 8,5, weiter bevorzugt bei pH 7,4. Zur Einstellung des pH-Werts werden Pufferlösungen wie beispielsweise Phosphatpuffer, Acetatpuffer, Acetat-boratpuffer, Citratpuffer und Boratpuffer verwendet.

Hyaluronsäure weist bzw. deren Hyaluronate weisen eine hohe Wasserbindungskapazität auf. Diese Wasserbindungskapazität bewirkt vorteilhaft, daß das Auge mit Feuchtigkeit versorgt bzw. daß einer Austrocknung des Auges entgegengewirkt wird. Darüber hinaus wirkt Hyaluronsäure bzw. wirken deren Hyaluronate auch als Viskositätsregler.

Als Viskositätsregler werden vorliegend Stoffe bezeichnet, die pharmakologisch verträglich sind und eine viskositätserhöhende Wirkung haben. Bevorzugt weisen die weiter verwendbaren Viskositätsregler ein viskoleastisches Verhalten auf.

Die viskositätserhöhende Wirkung bewirkt äußerst vorteilhaft, daß die auf die Augenoberfläche aufgebrachte pharmazeutische Zusammensetzung eine erhöhte Verweildauer aufweist und nicht von der Augenoberfläche umgehend wieder abfließt.

Neben der Hyaluronsäure bzw. dem Hyaluronat können zusätzlich auch Chondroitinsulfat, Polyacrylamid, Polyacrylsäure, Polyacrylharze, Polyethylenglykol, Cellulosederivate, Polyvinylalkohol, Polyvinylpyrrolidon oder Mischungen davon als Viskositätsregler verwendet werden.

Gemäß einer bevorzugten Ausführungsform wird neben Hyaluronsäure und/oder Hyaluronat kein weiterer Viskositätsregler verwendet.

Die Hyaluronsäure bzw. das Hyaluronat kann aus dem Glaskörper des Rinderauges oder aber auch aus Hahnenkämmen isoliert werden. Weiterhin kann Hyaluronsäure bzw. Hyaluronat auch in Bakterienstämmen in pharmazeutischer Qualität hergestellt werden.

Als Salze der Hyaluronsäure können beispielsweise Kalium-, Natrium-, Calcium- und/oder Magnesium-Hyaluronate verwendet werden.

Besonders bevorzugt ist das Hyaluronat Natrium-Hyaluronat.

Hyaluronsäure ist u. a. Bestandteil des Glaskörpers des Auges und stellt insofern keine für den menschlichen Organismus fremde Verbindung dar. Aus diesem Grund ist Hyaluronsäure aus immunologischer Sicht sehr gut verträglich.

Äußerst vorteilhaft weist Hyaluronsäure bzw. Hyaluronat eine strukturelle Ähnlichkeit mit Mucin auf. Mucin bildet die unterste Schicht des dreischichtigen Tränenfilms und sorgt für eine optimale Benetzung der Hornhaut- und Bindehautepithelien.

Die Hyaluronsäure und/oder das Hyaluronat imitiert damit die Schleimphase des Tränenfilms und verlängert zum einen die Verweildauer auf dem Auge und verbessert die Benetzbarkeit des Auges. Die Imitation der Schleimphase bewirkt zum anderen auch eine Verringerung der Reibung zwischen Auge und Augenlid und somit eine deutliche Abnahme einer mechanischen Reizung des Auges.

Die erfindungsgemäße Verwendung von Hyaluronsäure bzw. Hyaluronaten in der pharmazeutischen Zusammensetzung ist insbesondere bei Benetzungsstörungen des Auges, d.h. bei dem sogenannten "Trockenen Auge" und zur Behandlung von Epithelläsionen, die aus den Benetzungsstörungen resultieren, äußerst vorteilhaft.

Wässrige Natriumhyaluronatlösungen sind Flüssigkeiten mit nichtnewtonschen Fließeigenschaften. Aufgrund dieser physikalischen Eigenschaft eignen sich wässrige Natriumhyaluronatlösungen hervorragend als Gleit- und Schmiermittel mit guter Haftwirkung und verlängerter Verweilzeit auf den konjunktivalen und cornealen Epithelien ohne Beeinträchtigung der Sehleistung. Eine Konzentration von 0,1 Gew.-% von Natriumhyaluronat in der Zusammensetzung verbessert erheblich das bei der Behandlung von trockenen Augen wichtige subjektive Empfinden der Patienten.

Das nicht-newtonsche Fließverhalten der Hyaluronsäure bedingt eine für die Anwendung am Auge hervorragende Eigenschaft, daß nämlich die Viskosität mit zunehmender Schergeschwindigkeit abnimmt.

Nach Aufbringung der pharmazeutischen Zusammensetzung auf die Hornhaut des Auges wird über den Lidschlag des Augenlides eine Scherspannung an die pharmazeutische Zusammensetzung angelegt, wodurch die zunächst erhöhte Viskosität erniedrigt wird. Durch den Lidschlag des Augenlides erniedrigt sich die Viskosität, so daß sich ein gleichmäßiger Film auf der Oberfläche des Auges ausbildet. Nach dem Lidschlag erhöht sich die Viskosität, so daß der Film an der Augenoberfläche gut anhaftet.

Die weiterhin durch Hyaluronsäure bzw. Hyaluronat in zubereiteten Lösungen, Gelen, Pasten oder Salben bewirkten nichtnewtonschen Fließeigenschaften der pharmazeutischen Zusammensetzung und deren strukturelle Ähnlichkeiten zu Mucin bewirken neben einer hervorrragenden Gleit- und Schmierwirkung auch eine hervorragende Haftung an der Hornhaut des Auges. Die bei dem Sicca-Syndrom auftretende mechanische Reizung des Auges wird stark verringert bzw. beseitigt. Darüber hinaus wird durch die aufgrund der antinewtonschen Fließeigenschaften verbesserten Haftung der pharmazeutischen Zusammensetzung auf der Hornhaut des Auges eine raschere Heilung von Epithelläsionen bewirkt.

Weiterhin zeigen Natriumhyaluronat-haltige Augentropfen im Tierversuch wundheilungsfördernde Eigenschaften auf die Epithelien des Auges. Es wurde festgestellt, daß Hyaluronsäure bzw. Natriumhyaluronat konzentrationsabhängig die Migration von Epithelzellen und somit die Wundheilung fördert. Eine 0,1 Gew.-%ige Natriumhyaluronat-Lösung bewirkte bei Corneaepithelzellen von Kaninchen eine gesteigerte Epithelzellmigration.

Auch bewirkt Hyaluronsäure oder Natriumhyaluronat bei einer Verletzung des Corneaepithels oder bei einer Verätzung der Cornea eine raschere und bessere, d.h. unter weniger Narbenbildung ablaufende Wundheilung.

Der genaue Wirkmechanismus der Wundheilungsförderung durch Hyaluronsäure ist noch ungeklärt. Während eine Beeinflussung der Durchblutung der umgebenden Zellen als weniger wahrscheinlich erscheint, gibt es verschiedene Hinweise auf eine Wirkung auf Zellen, die im Entzündungsgeschehen eine Rolle spielen.

Schließlich zeigt Hyaluronsäure dosisabhängig eine schützende Wirkung vor Schädigung von Zellen durch Sauerstoffradikale. Freie Sauerstoffradikale verlangsamen die Wundheilung und spielen somit eine entscheidende Rolle im Entzündungsgeschehen.

Die antientzündliche Wirkung von Hyaluronsäure bzw. Hyaluronat sowie der durch Hyaluronsäure bzw. Hyaluronat vermittelte Schutz vor der schädlichen Wirkung von Sauerstoffradikalen wirken in synergistischer Weise mit der Wirkung von Panthenol oder Pantothensäure bei der erfindungsgemäßen Verwendung der hergestellten pharmazeutischen Zusammensetzung zusammen.

Gemäß einer weiter bevorzugten Ausführungsform weist die Hyaluronsäure und/oder das Hyaluronat ein Molekulargewicht auf, das in einem Bereich von etwa 50.000 bis etwa 10.000.000 Dalton, bevorzugt von etwa 250.000 bis etwa 5.000.000 Dalton, liegt. Besonders bevorzugt beträgt das Molekulargewicht der Hyaluronsäure bzw. des Hyaluronats 500.000 bis 4.000.000 Dalton. Äußerst bevorzugt weist die Hyaluronsäure bzw. das Hyaluronat ein Molekulargewicht von etwa 1.500.000 bis 3.500.000 Dalton auf.

Das hohe Molekulargewicht der Hyaluronsäure bzw. des verwendeten Hyaluronats wie beispielsweise Natriumhyaluronat bewirkt eine hohe Viskoelastizität bei niedriger Konzentration. In der Lösung liegen die Molekülketten in zufälliger Anordnung knäuelartig vor. Unter dem Einfluß der durch die Bewegung des Augenlides ausgeübten Scherkräfte richten sich die Makromoleküle in etwa parallel aus. Diese Änderung in der dreidimensionalen Struktur unter dem Einfluß von Scherkräften dürfte maßgeblich für die hervorragenden viskoelastischen Eigenschaften sein.

Bei Lidöffung überzieht die Substanz die Hornhautoberfläche und stellt aufgrund der hohen Wasserbindungskapazität von Hyaluronat auch einen Schutz gegen Verdunstung dar. Dies ist sowohl bei der Behandlung des Krankheitsbildes des "Trockenen Auges" von Vorteil, bei der es zu einer Verringerung der Menge an Tränenflüssigkeit im Auge kommt.

Gemäß einer weiteren bevorzugten Ausführungsform beträgt die Menge an Hyaluronsäure und/oder die Menge an Hyaluronat etwa 0,005 Gew.-% bis etwa 5 Gew.-%, bevorzugt etwa 0,01 Ges.-% bis etwa 1 Ges.-%, jeweils bezogen auf das Gesamtgewicht der pharmazeutischen Zusammensetzung.

Besonders bevorzugt beträgt die Menge an Hyaluronsäure und/oder die Menge an Hyaluronat etwa 0,05 Gew.-% bis etwa 0,5 Ges.-%, bezogen auf das Gesamtgewicht der pharmazeutischen Formulierung.

Äußerst vorteilhaft besitzt Hyaluronsäure bzw. besitzen Hyaluronate die Eigenschaft, Wasser zu binden. Diese Eigenschaft, Wasser zu binden, ist insbesondere bei der Behandlung des Sicca-Syndroms vorteilhaft, da der unerwünschten Austrocknung der Hornhaut des Auges entgegengewirkt wird. Konzentrationen von 0,1 Gew.-% bis 0,3 Gew.-%, bezogen auf das Gesamtgewicht der pharmazeutischen Formulierung, Hyaluronsäure und/oder Hyaluronat haben sich als sehr geeignet erwiesen.

Ein weiterer diagnostischer Parameter bei der Diagnose des Krankeitsbildes des "Trockenen Auges" ist die Tränenfilmaufreißzeit, die eine Aussage über die Qualität der Tränenflüssigkeit erlaubt. Dabei wird beispielsweise der Tränenfilm mit Fluorescein angefärbt und nachfolgend der Patient gebeten, die Augen möglichst lange ohne Blinkreflex offen zu halten. Unter Verwendung einer Spaltlampe wird dann festgestellt, wann der Tränenfilm zum ersten mal aufreißt. Liegt der Zeitraum unter 10 Sekunden, besteht der Verdacht auf das Krankheitsbild des "Trockenen Auges". Dabei hat sich Hyaluronsäure in einer Konzentration von 0,1 Gew.-% bis 0,3 Gew.-%, bezogen auf das Gesamtgewicht der pharmazeutischen Formulierung, im Hinblick auf die Verlängerung der Tränenfilmaufreißzeit als sehr wirksam erwiesen.

Die gleichzeitige Einwirkung von Panthenol und/oder Pantothensäure sowie die Bereitstellung eines künstlichen Tränenfilms führt zu einer synergistischen Wirkung, die eine raschere Therapie von Epithelläsionen, insbesondere beim Krankheitsbild des "Trockenen Auges", erlaubt.

Es hat sich überraschend gezeigt, daß die gemeinsame Applikation von Panthenol und/oder Pantothensäure und Hyaluronsäure und/oder Hyaluronat zu einer raschen Epithelialisierung führt. Zugleich wird der bei Epithelläsionen am Auge auftretende äußerst unangenehme Juckreiz rasch gelindert.

Die bei der erfindungsgemäßen Verwendung hergestellte pharmazeutische Zusammensetzung ist mithin eine Arzneistoffkombination.

Es hat sich gezeigt, daß die Hyaluronsäure und/oder das Hyaluronat aufgrund des nichtnewtonschen Fließverhaltens sehr geeignete viskoelastische Eigenschaften für die Verwendung auf der Augenoberfläche aufweisen. Das nichtnewtonsche Fließverhalten verzögert den Abfluß der am Auge aufgebrachten pharmazeutischen Zusammensetzung und verlängert somit den Kontakt mit der Hornhaut des Auges. Somit kann das Panthenol und/oder die Pantothensäure über einen längeren Zeitraum, beispielsweise von wenigstens 30 Minuten bis zu wenigstens 60 Minuten, auf der Cornea gehalten werden.

Durch die viskoelastischen Eigenschaften von Hyaluronsäure oder Hyaluronat wird das Panthenol und/oder die Pantothensäure bei jedem Lidschlag des Auges ohne weiteres wieder im wesentlichen gleichmäßig auf der gesamten Augenoberfläche verteilt, sofern es zu einem gewissen Abfluß gekommen sein sollte.

Äußerst vorteilhaft wirkt damit das Panthenol und/oder die Pantothensäure über die gesamte Behandlungsdauer gleichmäßig auf die Augenoberfläche ein. Hierdurch kann vorteilhaft bei der Therapie zum einen die Dosierung von Panthenol und/oder Pantothensäure verringert werden und zum anderen kann die Behandlungsdauer verkürzt werden.

Bevorzugt ist, daß die Menge an Panthenol und/oder die Pantothensäure etwa 0,5 Gew.-% bis 10 Gew.-%, bevorzugt etwa 2 Gew.-% bis 5 Gew.-%, bezogen auf das Gesamtgewicht der pharmazeutischen Zusammensetzung beträgt.

Als sehr geeignet hat sich eine Menge von etwa 3 Gew.-%, bezogen auf das Gesamtgewicht der pharmazeutischen Zusammensetzung, erwiesen.

Gemäß einer bevorzugten Ausführungsform liegt das Panthenol als D-(+)-2,4-Dihydroxy-N-(3-hydroxypropyl)-3,3-dimethylbutyramid vor. Diese rechtsdrehende D-Konfiguration wird auch als Dexpanthenol bezeichnet.

Gemäß einer weiteren bevorzugten Ausführungsform der Erfindung liegt die Pantothensäure als wasserlösliches Salz, bevorzugt als Natriumpantothenat oder Calciumpantothenat, vor.

Äußerst vorteilhaft weist Dexpanthenol wasserbindende Eigenschaften auf. Hierdurch werden vorteilhaft die wasserbindenden Eigenschaften von Hyaluronsäure und/oder Hyaluronat ergänzt. Darüber hinaus ergänzen sich in bislang nicht verstandener Weise die wundheilungsfördernden Wirkungen von Hyaluronsäure und/oder Hyaluronat und von Pantothensäure und/oder Panthenol, insbesondere von Dexpanthenol bei Epithelläsionen des Hornhautepitheliums.

Weiterhin ist bevorzugt, daß die Zusammensetzung in Form einer Lösung, Suspension, Emulsion, eines Gels, einer Salbe, Paste, eines Pulvers, Granulats oder einer Tablette vorliegt, die bevorzugt direkt am Auge verwendet oder auf die Augenoberfläche aufgebracht werden kann.

Gemäß einer bevorzugten Ausführungsform liegt die pharmazeutische Zusammensetzung in der Form einer Lösung vor, so daß diese beispielsweise in der Form von Augentropfen oder eines Augensprays auf die Hornhautoberfläche des Auges aufgebracht werden kann.

Selbstverständlich ist es möglich, daß die pharmazeutische Zusammensetzung in der Form eines Feststoffes vorliegt, der vor einer Applikation zunächst in einer wäßrigen Lösung, wie beispielsweise einer Pufferlösung, aufgelöst wird. Nach Auflösung eines Feststoffes, beispielsweise in einer wäßrigen Pufferlösung, kann die Lösung sterilfiltriert und dann als Augenspray bzw. Augentropfen auf die Cornea aufgebracht werden. Bevorzugt liegen Feststoff und Lösungsmittel bei getrennter Aufbewahrung bereits in steriler Form vor, so daß ein Sterilfiltrieren nach Herstellung der Lösung nicht erforderlich ist. Der Anwender kann somit nach Herstellung der Mischung bzw. Lösung die pharmazeutische Zusammensetzung direkt applizieren.

Bei Bereitstellung der pharmazeutischen Zusammensetzung in Form eines Feststoffs wie beispielsweise eines Pulvers, Puders, Granulats oder einer Tablette umfaßt die pharmazeutische Zusammensetzung bevorzugt Panthenol, Pantothensäure und/oder Salze der Pantothensäure, welche ohne weiteres in wässrigen Lösungen gelöst werden können, sowie die in Wasser sehr gut lösliche Hyaluronsäure und/oder das in Wasser sehr gut lösliche Natriumhyaluronat.

Bevorzugt liegt die bei der erfindungsgemäßen Verwendung hergestellte pharmazeutische Zusammensetzung in steriler Form in Einzeldosis- oder Mehrfachdosenbehältnissen vor.

Bevorzugt wird zur Lagerung und Abgabe einer konservierungsmittelfreien pharmazeutischen Zusammensetzung das in "PTA heute" 1996, Nr. 12, S. 1230-1232 beschriebene COMOD^{®}-System, verwendet, das eine sterile Lagerung und Mehrfachabgabe der pharmazeutischen Zusammensetzung erlaubt. Selbstverständlich können auch herkömmliche Einzeldosisbehältnisse verwendet werden, die nach Gebrauch weggeworfen werden.

Bei Verwendung von Hyaluronsäure und/oder Hyaluronat in der pharmazeutischen Zusammensetzung wird die pharmazeutische Zusammensetzung bevorzugt konservierungsmittelfrei bereitgestellt.

Konservierungsmittel können den präcornealen Tränenfilm schädigen und zu einer Reduzierung der Anzahl der Mikrovilli und Mikroplicae der oberflächlichen Hornhautepitelzellen führen. Insbesondere besitzt das weit verbreitete Benzalkoniumchlorid eine große Schädigungspotenz. Im Hinblick auf die gewünschte Therapie einer durch das Sicca-Syndrom bedingten Reizung des Auges ist es vorteilhaft, jede weitere Reizung und/oder Schädigung des Auges durch den Zusatz von Konservierungsmittel zu vermeiden.

Grundsätzlich kann die bei der erfindungsgemäßen Verwendung hergestellte pharmazeutische Zusammensetzung auch in Form von Augentabletten in den Bindehautsack eingebracht werden. Unter Einwirkung von Tränenflüssigkeit löst sich die Augentablette rasch auf.

Bevorzugt erfolgt jedoch die Applikation der pharmazeutischen Zusammensetzung am Auge in der Form von Augentropfen.

Bei Bereitstellung der pharmazeutischen Zusammensetzung in Form von Augensalben bzw. Augengelen werden die Wirkstoffe beispielsweise in Vaselin oder Paraffin mit und ohne Emulgatorzusatz wie beispielsweise Cholesterin, Wollwachs, Wollwachsalkohole, Cetanol, etc. bereitgestellt.

Bevorzugt wird die pharmazeutische Zusammensetzung zur Behandlung von ophthalmologischen Fehlfunktionen, die mit Benetzungsstörungen der Hornhaut des Auges verbunden ist, verwendet.

Weiter bevorzugt wird die pharmazeutische Zusammensetzung zur Behandlung von allergischer Rhinokonjunktivitis, atopischer Keratokonjunktivitis, allergischer Keratokonjunktivitis, gigantopapillärer Konjunktivitis, Konjunktivitis vernalis, Episkleritis, wie beispielsweise Episcleritis periodica, Episcleritis partialis fugax, Skleritis, Tenonitis, Sjögren-Syndrom oder Mischformen davon verwendet.

Da das Auge für den Menschen ein wichtiges Sinnesorgan ist, stellt die bei der erfindungsgemäßen Verwendung hergestellte pharmazeutische Zusammensetzung einen bedeutenden Fortschritt auf dem Gebiet der Ophthalmologie dar.

### Beispiel

### Pharmazeutische Zusammensetzung zur ophthalmologischen Applikation

| | |
|---|---|
| 50 mg/ml | Dexpanthenol |
| 1,55 mg/ml | Hyaluronsäure, Molekulargewicht: 1,5 x 10⁶ - 3,5 x 10⁶ Dalton |
| 2 mg/ml | Natriumcitrat |
| Zugabe von 1 %-iger wässriger Citronensäurelösung bis pH 7,0 - pH 7,4 ereicht ist | |
| Zugabe von Wasser für Injektionszwecke ad 1 ml | |

## Patentansprüche

1. Verwendung von Panthenol und/oder Pantothensäure und Hyaluronsäure und/oder Hyaluronat sowie gegebenenfalls zusätzlichen pharmazeutischen Hilfsmitteln zur Herstellung einer pharmazeutischen Zusammensetzung zur Behandlung ophtalmologischer Fehlfunktionen.

2. Verwendung nach Anspruch 1,
**dadurch gekennzeichnet,**
**daß** die Menge an Hyaluronsäure und/oder die Menge an Hyaluronat etwa 0,005 Gew.-% bis etwa 5 Ges.-%, bevorzugt etwa 0,01 Gew.-% bis etwa 1 Ges.-%, jeweils bezogen auf das Gesamtgewicht der pharmazeutischen Zusammensetzung, beträgt.

3. Verwendung nach Anspruch 1 oder 2,
**dadurch gekennzeichnet,**
**daß** die Hyaluronsäure und/oder das Hyaluronat ein Molekulargewicht aufweist, das in einem Bereich von etwa 50.000 bis etwa 10.000.000 Dalton, bevorzugt von etwa 250.000 bis etwa 5.000.000 Dalton, liegt.

4. Verwendung nach einem der Ansprüche 1 bis 3,
**dadurch gekennzeichnet,**
**daß** das Hyaluronat Natrium-Hyaluronat ist.

5. Verwendung nach einem der Ansprüche 1 bis 4,
**dadurch gekennzeichnet,**
**daß** die Menge an Panthenol und/oder die Pantothensäure etwa 0,5 Gew.-% bis 10 Gew.-%, bevorzugt etwa 2 Gew.-% bis 5 Gew.-%, bezogen auf das Gesamtgewicht der pharmazeutischen Zusammensetzung beträgt.

6. Verwendung nach einem der Ansprüche 1 bis 5,
**dadurch gekennzeichnet,**
**daß** das Panthenol als Dexpanthenol vorliegt.

7. Verwendung nach einem der Ansprüche 1 bis 5,
**dadurch gekennzeichnet,**
**daß** die Pantothensäure als wasserlösliches Salz, bevorzugt als Natriumpantothenat oder Calciumpantothenat, vorliegt.

8. Verwendung nach einem der Ansprüche 1 bis 7,
**dadurch gekennzeichnet,**
**daß** die pharmazeutische Zusammensetzung in Form einer Lösung, Suspension, Emulsion, eines Gels, einer Salbe, Paste, eines Puders, Pulvers, Granulats oder einer Tablette vorliegt.

9. Verwendung nach einem der Ansprüche 1 bis 8,
**dadurch gekennzeichnet,**
**daß** die ophthalmologische Fehlfunktion mit Benetzungsstörungen der Hornhaut und Bindehaut des Auges verbunden ist.

10. Verwendung nach einem der Ansprüche 1 bis 9,
**dadurch gekennzeichnet,**
**daß** die ophthalmologische Fehlfunktion aus der Gruppe ausgewählt wird, die aus allergischer Rhinokonjunktivitis, atopischer Keratokonjunktivitis, allergischer Keratokonjunktivitis, gigantopapilläre Konjunktivitis, Konjunktivitis vernalis, Episkleritis, Skleritis, Tenonitis, Sjögren-Syndrom und Mischformen davon besteht.

## Claims

1. Use of Panthenol and/or pantothenic acid and hyaluronic acid and/or hyaluronate and optionally additional pharmaceutical auxiliary agents for the production of a pharmaceutical composition for the treatment of ophthalmological malfunctions.

2. Use according to claim 1,
**characterised in that**
the amount of hyaluronic acid and/or the amount of hyaluronate is about 0.005% by weight to about 5% by weight, preferably about 0.01% by weight to about 1% by weight with respect to the total weight of the pharmaceutical composition.

3. Use according to claim 1 or claim 2,
**characterised in that**
the hyaluronic acid and/or the hyaluronate has a molecular weight which is in a range of about 50,000 to about 10,000,000 Daltons, preferably about 250,000 to about 5,000,000 Daltons.

4. Use according to one of claims 1 to 3,
**characterised in that**
the hyaluronate is sodium hyaluronate.

5. Use according to one of claims 1 to 4,
**characterised in that**
the amount of panthenol and/or pantothenic acid is about 0.5% by weight to 10% by weight, preferably about 2% by weight to 5% by weight with respect to the total weight of the pharmaceutical composition.

6. Use according to one of claims 1 to 5,
**characterised in that**
the panthenol is in the form of dexpanthenol.

7. Use according to one of claims 1 to 5,
**characterised in that**
the pantothenic acid is in the form of a watersoluble salt, preferably sodium pantothenate or calcium pantothenate.

8. Use according to one of claims 1 to 7,
**characterised in that**
the pharmaceutical composition is in the form of a solution, suspension, emulsion, gel, ointment, paste, powder, particles, granules or a tablet.

9. Use according to one of claims 1 to 8,
**characterised in that**
the ophthalmological malfunction is linked to disturbances to wetting of the cornea and conjunctiva of the eye.

10. Use according to one of claims 1 to 9,
**characterised in that**
the ophthalmological malfunction is selected from the group consisting of allergic rhinoconjunctivitis, atopic keratoconjunctivitis, allergic keratoconjunctivitis, gigantopapillary conjunctivitis, vernal conjunctivitis, episcleritis, scleritis, tenonitis, Sjögren's syndrome and hybrid forms thereof.

## Revendications

1. Utilisation de panthénol et/ou d'acide pantothénique et d'acide hyaluronique et/ou de hyaluronate, ainsi que, éventuellement, des auxiliaires pharmaceutiques supplémentaires pour la fabrication d'une composition pharmaceutique pour le traitement de dysfonctionnements ophtalmologiques.

2. Utilisation selon la revendication 1, **caractérisée en ce que** la quantité d'acide hyaluronique et/ou la quantité de hyaluronate est d'environ 0,005 % en poids à environ 5 % en poids, de préférence d'environ 0,01 % en poids à environ 1 % en poids, respectivement, par rapport au poids total de la composition pharmaceutique.

3. Utilisation selon la revendication 1 ou 2, **caractérisée en ce que** l'acide hyaluronique et/ou le hyaluronate présente un poids moléculaire qui se situe dans une plage d'environ 50 000 à environ 10 000 000 Dalton, de préférence dans une plage d'environ 250 000 à environ 5 000 000 Dalton.

4. Utilisation selon l'une quelconque des revendications 1 à 3, **caractérisée en ce que** le hyaluronate est le hyaluronate de sodium.

5. Utilisation selon l'une quelconque des revendications 1 à 4, **caractérisée en ce que** la quantité de panthénol et/ou l'acide pantothénique est d'environ 0,5 % en poids à 10 % en poids, de préférence d'environ 2 % en poids à environ 5 % en poids, par rapport au poids total de la composition pharmaceutique.

6. Utilisation selon l'une quelconque des revendications 1 à 5, **caractérisée en ce que** le panthénol se présente sous forme de dexpanthénol.

7. Utilisation selon l'une quelconque des revendications 1 à 5, **caractérisée en ce que** l'acide pantothénique se présente sous forme de sel hydrosoluble, de préférence sous forme de pantothénate de sodium ou de pantothénate de calcium.

8. Utilisation selon l'une quelconque des revendications 1 à 7, **caractérisée en ce que** la composition pharmaceutique se présente sous la forme d'une solution, d'une suspension, d'une émulsion, d'un gel, d'une pommade, d'une pâte, d'une poudre, de granulés ou d'un comprimé.

9. Utilisation selon l'une quelconque des revendications 1 à 8, **caractérisée en ce que** le dysfonctionnement ophtalmologique est lié à des troubles de mouillage de la cornée et de la conjonctive de l'oeil.

10. Utilisation selon l'une quelconque des revendications 1 à 9, **caractérisée en ce que** le dysfonctionnement ophtalmologique est choisi dans le groupe comprenant la rhino-conjonctivite allergique, la kérato-conjonctivite atopique, la kéroto-conjonctivite allergique, la conjonctivite giganto-papillaire, la conjonctivite printanière, l'épisclérite, la sclérite, la ténonite, le syndrome de Sjören et des formes mixtes de ceux-ci.
